# EUROPEAN PATENT APPLICATION

(11) **EP 2 679 163 A1**
(43) Date of publication of application: **01.01.2014**
(21) Application number: 13168908.5
(22) Date of filing: 23.05.2013
(51) Int. Cl.: A61B 8/00, G01S 7/52, A61B 8/08

(54) **Ultrasound image displaying method using marker and ultrasound diagnosis apparatus**

(30) Priority: 28.06.2012 KR 20120070233
(71) Applicant: Samsung Medison Co., Ltd., Gangwon-do 250-870 (KR)
(72) Inventor: Lee, Jin-yong, Gangwon-do (KR); Lee, Bong-heon, Gangwon-do (KR); Chang, Eun-jung, Gangwon-do (KR)
(74) Representative: Lorenz, Markus

(57) **Abstract**

An ultrasound image displaying method and an ultrasound diagnosis apparatus include: displaying a first ultrasound image obtained from an object in a first mode and a second ultrasound image obtained from the object in a second mode; displaying a first marker and a second marker indicating locations of parts of the object on the first ultrasound image and the second ultrasound image, respectively; and as the first marker moves based on an external input signal, moving the second marker such that the second marker matches with the moved first marker.

## Description

### CROSS-REFERENCE TO RELATED PATENT APPLICATION

This application claims the benefit of Korean Patent Application No. 10-2012-0070233, filed on June 28, 2012, in the Korean Intellectual Property Office, the disclosure of which is incorporated herein in its entirety by reference.

### BACKGROUND OF THE INVENTION

### 1. Field of the Invention

The present invention relates to an ultrasound image displaying method and apparatus for obtaining an exact data value with respect to an ultrasound image displayed on an ultrasound diagnosis apparatus.

### 2. Description of the Related Art

Ultrasound diagnosis apparatuses generate an ultrasound signal (generally greater than 20 kHz) regarding a predetermined part inside an object by using a probe, and obtain an image of the part inside the object by using information regarding a reflected echo signal. In particular, ultrasound diagnosis apparatuses are used for medical purposes, such as detection of impurities inside the object, measurement and observation of a wound thereof, etc. Such ultrasound diagnosis apparatuses have various advantages, including real-time display and safety due to there being no radioactive exposure compared to X-ray apparatuses, and thus, the ultrasound diagnosis apparatuses are commonly used together with other image diagnosis apparatuses.

An image (hereinafter referred to as an ultrasound image) obtained through ultrasound diagnosis apparatuses may be displayed on the ultrasound diagnosis apparatuses or may be stored in a storage medium and displayed on other ultrasound diagnosis apparatuses. For example, the ultrasound image may be reduced and displayed on screens of cellular phones, portable electronic devices, personal digital assistants (PDAs), tablet PCs, etc.

### SUMMARY OF THE INVENTION

When conventional ultrasound diagnosis technology compares corresponding data values in two or more pieces of displayed data, a user personally compares the data values by using markings or numbers. Thus, the conventional technology involves the inconvenient process of comparing the data values by the user, and it is difficult to obtain an exact comparison result. The present invention provides a method and apparatus to solve these problems of the conventional technology. The present invention also provides a computer readable recording medium having embodied thereon a computer program for executing the method.

According to an aspect of the present invention, there is provided an ultrasound image displaying method including: displaying a first ultrasound image obtained from an object in a first mode and a second ultrasound image obtained from the object in a second mode; displaying a first marker and a second marker indicating locations of parts of the object on the first ultrasound image and the second ultrasound image, respectively; and as the first marker moves based on an external input signal, moving the second marker such that the second marker matches with the moved first marker.

The first mode may include a brightness (B) mode, and the second mode includes a motion (M) mode.

A depth axis of the second ultrasound image may match with one of scan lines included in the first ultrasound image.

The external input signal may move a depth value of the first marker, wherein the moving of the second marker includes: moving a depth value of the second marker such that the depth value of the second marker matches with the moved depth value of the first marker.

The first ultrasound image may be a two-dimensional (2D) image or a three-dimensional (3D) image obtained by scanning the object.

According to another aspect of the present invention, there is provided an ultrasound image displaying method including: displaying a volume data image obtained from an object and a planar image obtained from the object; displaying a first marker and a second marker indicating locations of parts of the object on the volume data image and the planar image, respectively; and as the first marker moves based on an external input signal, moving the second marker such that the second marker matches with the moved first marker.

The planar image may include a plurality of planar images, wherein the displaying of the first marker and the second marker includes: displaying a plurality of first markers on the volume data image; and displaying a plurality of second markers on the plurality of planar images.

The plurality of planar images may include at least one of an A plane image, a B plane image, and a C plane image obtained by cutting the volume data image in previously determined directions.

The displaying of the plurality of planar images may include: displaying the A plane image, the B plane image, and the C plane image, wherein the displaying of the plurality of second markers includes: displaying the plurality of second markers on the A plane image, the B plane image, and the C plane image, wherein the displaying of the plurality of first markers includes: displaying the plurality of first markers, respectively, matching with the second marker displayed on the A plane image, the second marker displayed on the B plane image, and the second marker displayed on the C plane image, on the volume data image.

The displaying of the plurality of second markers may include: visually distinguishing and displaying the plurality of second markers for each of the plurality of planar images.

The displaying of the volume data image may include: displaying a cutting surface corresponding to the planar image and the volume data image together, wherein the moving of the first marker includes: moving the first marker on the cutting surface.

According to another aspect of the present invention, there is provided an ultrasound diagnosis apparatus for displaying an ultrasound image, the apparatus including: an ultrasound image obtaining unit for obtaining a first ultrasound image from an object in a first mode and obtaining a second ultrasound image from the object in a second mode; a display unit for displaying the first ultrasound image and the second ultrasound image and displaying a first marker and a second marker indicating locations of parts of the object on the first ultrasound image and the second ultrasound image, respectively; and an external input receiving unit for receiving an external input signal, wherein the display unit, as the first marker moves based on the external input signal, moves the second marker such that the second marker matches with the moved first marker.

According to another aspect of the present invention, there is provided an ultrasound diagnosis apparatus for displaying an ultrasound image, the apparatus including: an ultrasound image obtaining unit for obtaining a volume data image and a planar image from an object; a display unit for displaying the volume data image and the planar image, and displaying a first marker and a second marker indicating locations of parts of the object on the volume data image and the planar image, respectively; and an external input receiving unit for receiving an external input signal, wherein the display unit, as the first marker moves based on the external input signal, moves the second marker such that the second marker matches with the moved first marker.

According to another aspect of the present invention, there is provided a computer readable recording medium having embodied thereon a computer program for executing the ultrasound image displaying method.

According to another aspect of the present invention, there is provided a computer readable recording medium having embodied thereon a computer program for executing the ultrasound image displaying method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an ultrasound diagnosis apparatus according to an embodiment of the present invention;
FIG. 2 is a flowchart illustrating an ultrasound image displaying method according to an embodiment of the present invention;
FIG. 3 shows an embodiment in which a first ultrasound image and a second ultrasound image are displayed and markers displayed on the first ultrasound image and the second ultrasound image move according to an embodiment of the present invention;
FIG. 4 shows an embodiment of a volume data image and a planar image;
   and
FIG. 5 shows an embodiment of a volume data image and a plurality of planar images.

### DETAILED DESCRIPTION OF THE INVENTION

Most of the terms used herein are general terms that have been widely used in the technical art to which the present invention pertains. However, some of the terms used herein may be created reflecting intentions of technicians in this art, precedents, or new technologies. Also, some of the terms used herein may be arbitrarily chosen by the present applicant. In this case, these terms are defined in detail below. Accordingly, the specific terms used herein should be understood based on the unique meanings thereof and the whole context of the present invention.

In the present specification, it should be understood that the terms, such as 'including' or 'having,' etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Also, the terms, such as 'unit' or 'module', etc., should be understood as a unit that processes at least one function or operation and that may be embodied in a hardware manner, a software manner, or a combination of the hardware manner and the software manner. As used herein, expressions such as "at least one of," when preceding a list of elements, modify the entire list of elements and do not modify the individual elements of the list.

The embodiments of the present invention will now be described more fully with reference to the accompanying drawings.

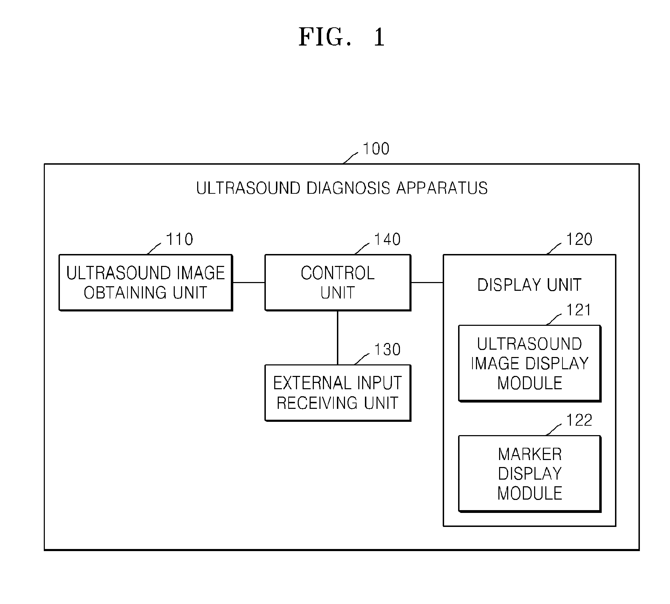

FIG. 1 is a block diagram of an ultrasound diagnosis apparatus 100 according to an embodiment of the present invention.

Referring to FIG. 1, the ultrasound diagnosis apparatus 100 according to an embodiment of the present invention may include an ultrasound image obtaining unit 110, a display unit 120, an external input receiving unit 130, and a control unit 140.

The ultrasound image obtaining unit 110 scans an object and obtains an ultrasound image. The ultrasound image obtaining unit 110 may include a probe for scanning the object, for example, a two-dimensional (2D) probe or a three-dimensional (3D) probe.

The ultrasound image obtaining unit 110 may scan the object in a mode among an amplitude (A) mode, a brightness (B) mode, and a motion (M) mode by using the probe. The A mode indicates intensity of an echo signal according to a distance from the ultrasound image obtaining unit 110 to the object. The B mode indicates brightness representing the measured intensity of the echo signal. The M mode indicates a variation with respect to time of the B mode.

The ultrasound image obtaining unit 110 may scan the object through various types of methods and modes besides the above-described A mode, B mode, and M mode, and obtain the ultrasound image.

The ultrasound image obtained by scanning the object by the ultrasound image obtaining unit 110 may include volume data that are a 2D image and a 3D image, and an image of a plane of the volume data. That is, the ultrasound image obtaining unit 110 may obtain a 2D image of the object, the volume data by using the 2D probe or the 3D probe, and an image of a plane of the obtained volume data.

The planar image obtained by the ultrasound image obtaining unit 110 may be an image of a plane obtained by cutting the volume data in a previously determined direction. For example, the planar image may include an A plane image taken from an axial view, a B plane image taken from a sagittal view, and a C plane image taken from a coronal view.

Although the ultrasound image obtaining unit 110 may scan the target boy and obtain the ultrasound image as described above, the ultrasound image obtaining unit 110 may obtain the ultrasound image from a storage unit (not shown) included therein. That is, the ultrasound image obtaining unit 110 may receive the ultrasound image from an externally connected storage medium and store the ultrasound image in the storage unit (not shown) or may obtain the ultrasound image from a local server through a picture archiving communication system (PACS). The storage unit (not shown) may store the ultrasound image obtained by wire or wirelessly as described above. The ultrasound image obtaining unit 110 may obtain the ultrasound image from the storage unit (not shown).

The display unit 120 displays the obtained ultrasound image. Also, the display unit 120 displays a marker indicating a location of a part of the object on the ultrasound image.

The display unit 120 may include at least one of a liquid crystal display (LCD), a thin film transistor liquid crystal display (TFT-LCD), an organic light emitting diode (OLED), a flexible display, and a 3D display. Also, the ultrasound diagnosis apparatus 100 may include two or more display units 140 according to an implementation shape thereof.

According to an embodiment, the display unit 120 may be a touch screen that includes an external input receiving unit 130 and a layer structure. That is, the display unit 120 may be used as both an output apparatus and an input apparatus. In this case, the display unit 120 may receive a touch input using a stylus pen or a part of the human body.

Also, in a case where the display unit 120 is the touch screen including the layer structure as described above, the display unit 120 may detect a touch input location, a touch input area, a touch pressure, etc. Also, the touch screen may detect a real touch as well as a proximity touch.

The display unit 120 may include an ultrasound image display module 121 and a marker display module 122. The ultrasound image display module 121 may display the ultrasound image obtained by the ultrasound image obtaining unit 110. That is, the ultrasound image display module 121 may display at least one of a 2D ultrasound image, a volume data image, and a planar image of the volume data image.

The market display module 122 may overlay and display one or more markers on the displayed ultrasound image. That is, the marker display module 122 may display the marker indicating a location of a part of the object on the displayed ultrasound image.

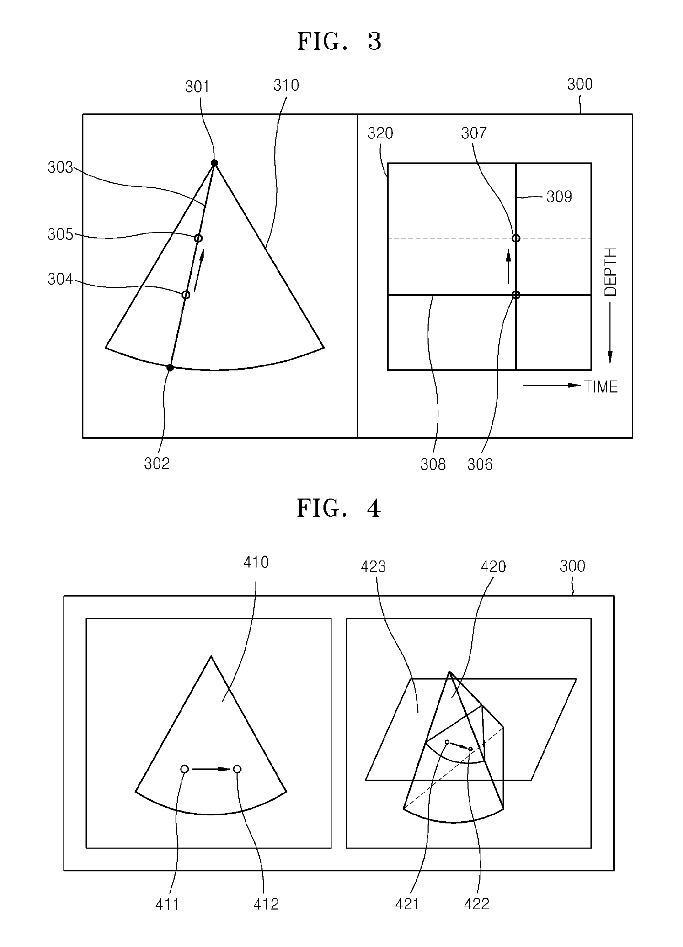

Also, the marker display module 122 may move and display the marker. That is, in a case where the marker is displayed on the ultrasound image, the marker display module 122 may move a location of the displayed marker based on an external input signal. The location of the marker may move according to a scan line of the ultrasound image or a depth axis or on a cut plane of the volume data. This will be described in detail with reference to FIGS. 3 through 5.

Also, in a case where the ultrasound image display module 121 displays a plurality of planar images, the marker display module 122 may visually distinguish and display a plurality of markers on the planar images. That is, the marker display module 122 may display one or more visually distinguished markers such as '○', 'X', and '△' on the planar images.

The external input receiving unit 130 receives an input from the outside of the ultrasound diagnosis apparatus 100 by wire or wirelessly. That is, the external input receiving unit 130 may receive a user input from a user of the ultrasound diagnosis apparatus 100 or a control signal from a device other than the ultrasound diagnosis apparatus 100.

The external input receiving unit 130 may include a mouse, a key pad, and a track ball that receive a user input. Alternatively, in a case where the display unit 120 that is the touch screen is used as both the output apparatus and the input apparatus as described above, the external input receiving unit 130 may be implemented by being included in the display unit 120.

The external input receiving unit 130 may receive an external input used to move the marker displayed on the ultrasound image. This will be described in detail later with reference to FIGS. 3 through 5.

The control unit 140 generally controls the ultrasound image obtaining unit 110, the display unit 120, and the external input receiving unit 130. More specifically, the control unit 140 may control the display unit 120 to display the ultrasound image obtained by the ultrasound image obtaining unit 110. Also, the control unit 140 may control the display unit 120 to display the marker on the ultrasound image, and may control the external input receiving unit 130 to move the displayed marker.

Only the elements related to the present embodiment are shown in FIG. 1. It will be understood by those of ordinary skill in the art that the ultrasound diagnosis apparatus 100 may further include other general-purpose elements, in addition to the elements.

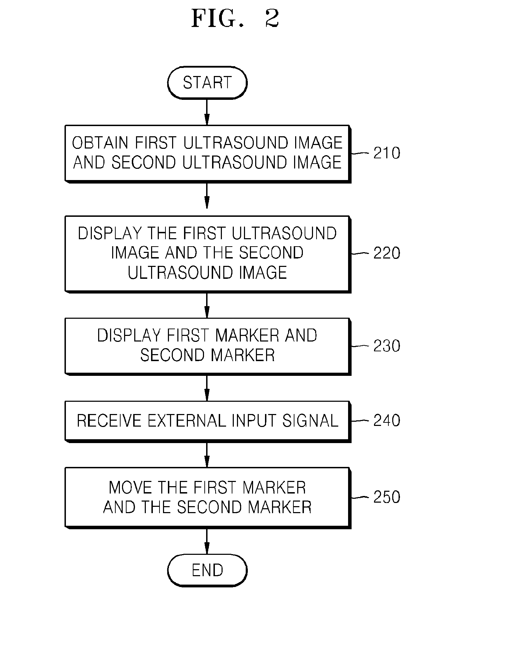

FIG. 2 is a flowchart illustrating an ultrasound image displaying method using the elements included in the ultrasound diagnosis apparatus 100 according to an embodiment of the present invention. The method of FIG. 2 includes operations time-sequentially performed by the ultrasound diagnosis apparatus 100, the ultrasound image obtaining unit 110, the display unit 120, and the external input receiving unit 130, and the control unit 140 of FIG. 1. Thus, although omitted below, the descriptions of the elements of FIG. 1 may also apply to the method of FIG. 2.

In operation 210, the ultrasound diagnosis apparatus 100 obtains a first ultrasound image and a second ultrasound image. More specifically, the ultrasound diagnosis apparatus 100 may scan an object and obtain the first ultrasound image and the second ultrasound image, and, as described above, may receive and obtain an ultrasound image from a storage medium connected to the ultrasound diagnosis apparatus 100 or from an external server.

The first ultrasound image and the second ultrasound image obtained in operation 210 may be 2D images, volume data images, or planar images of volume data obtained by scanning the object in one of an A mode, a B mode, and a C mode.

In operation 220, the ultrasound diagnosis apparatus 100 may match and display the first ultrasound image and the second ultrasound image obtained in operation 210.

According to an embodiment, the ultrasound diagnosis apparatus 100 may match and display a 2D image of the B mode and a 2D image of the M mode. More specifically, the ultrasound diagnosis apparatus 100 may match and display the 2D image of the B mode and a 2D image of the M mode indicating a time variation with respect to one of scan lines included in the 2D image of the B mode. This will be described in detail with reference to FIG. 3.

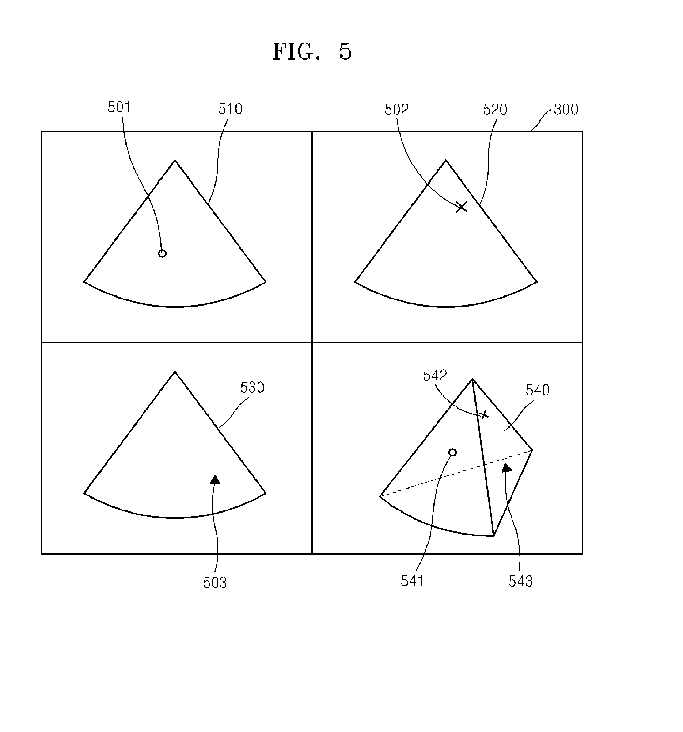

According to another embodiment, the ultrasound diagnosis apparatus 100 may match and display the volume data image and the planar image of the volume data. This will be described in detail with reference to FIGS. 4 and 5.

In operation 230, the ultrasound diagnosis apparatus 100 displays a first marker and a second marker. The first marker and the second marker may spatially match and be displayed in operation 230. In other words, the ultrasound diagnosis apparatus 100 may display the spatially matching first marker and second marker on each of the first ultrasound image and the second ultrasound image displayed in operation 220.

According to an embodiment, the volume data image, an image of an A plane, an image of a B plane, and an image of a C plane may match and be displayed in operation 220. In this regard, spatially matching markers may be displayed on each of the image of the A plane, the image of the B plane, and the image of the C plane, and the volume data image in operation 230.

In other words, matching markers may be displayed on each of the image of the A plane and the volume data image, matching markers may be displayed on each of the image of the B plane and the volume data image, and matching markers may be displayed on each of the image of the C plane and the volume data image.

In accordance with the above embodiment, one marker may be displayed on each of the image of the A plane, the image of the B plane, and the image of the C plane, and three markers matching the A plane image, the B plane image, and the C plane image may be displayed on the volume data. In this regard, the ultrasound diagnosis apparatus 100 may visually distinguish and display the three markers displayed on the volume data.

In operation 240, the ultrasound diagnosis apparatus 100 receives an external input signal. The external input signal received in operation 240 may be a signal for moving at least one of the markers including the first marker and the second marker displayed in operation 230. That is, the ultrasound diagnosis apparatus 100 may receive the external input signal for moving one of the first marker and the second marker.

According to an embodiment, the ultrasound diagnosis apparatus 100 may receive a touch input received through a touch screen, a drag and drop input, and a swipe input as the external input signal. Meanwhile, the external input signal may be received through a track ball.

In operation 250, the ultrasound diagnosis apparatus 100 moves the first marker and the second marker. That is, the ultrasound diagnosis apparatus 100 moves spatially matching markers based on the external input signal received in operation 240.

According to an embodiment, in a case where the external input signal for moving the first marker is received in operation 240, the ultrasound diagnosis apparatus 100 may move the first marker as well as the second marker that spatially matches with the first marker in operation 250. In other words, the first marker and the second marker move together according to a matching relationship based on the external input signal for moving one of the first marker and the second marker.

Throughout operation 250 and the specification below, the first marker and the second marker are determined according to a movement sequence of the first marker and the second marker. That is, the first marker and the second marker are not distinguished according to images on which the first marker and the second marker are displayed but a marker that moves earlier according to the external input signal is determined as the first marker, and a marker that moves later is determined as the second marker.

That is, if a marker displayed on a first image moves earlier, the marker may be determined as the first marker, and a marker displayed on a second image may be determined as the second marker. To the contrary, if the marker displayed on the second image moves earlier, the marker may be determined as the first marker, and the marker displayed on the first image may be determined as the second marker.

A user may exactly measure data values of matching ultrasound images by using the above-described ultrasound image displaying method. That is, the first marker and the second marker move together according to the matching relationship, thereby easily and exactly detecting a corresponding point of two ultrasound images. Accordingly, a user of the ultrasound diagnosis apparatus 100 may exactly diagnose the object.

FIG. 3 shows an embodiment in which a first ultrasound image 310 and a second ultrasound image 320 are displayed and markers displayed on the first ultrasound image 310 and the second ultrasound image 320 move according to an embodiment of the present invention.

In the present embodiment, the display unit 120 displays the first ultrasound image 310 that is a 2D ultrasound image of a B mode and the second ultrasound image 320 that is a 2D ultrasound image of an M mode on a screen 300 of the ultrasound diagnosis apparatus 100.

The first ultrasound image 310 may be an image obtained by scanning an object along a plurality of scan lines. Also, the second ultrasound image 320 that is an image of the M mode may be an image obtained according to a time variation with respect to one of the scan lines included in the first ultrasound image 310. That is, a horizontal axis of the second ultrasound image 320 may indicate time, and a vertical axis thereof may indicate a depth.

In the present embodiment, the ultrasound diagnosis apparatus 100 may match and display the second ultrasound image 320 and an image of the B mode that is the first ultrasound image 310. That is, the ultrasound diagnosis apparatus 100 may match and display a scan line 303 connected from a point 301 to a point 302 among the plurality of scan lines and a depth axis 309 of the image of the M mode.

The ultrasound diagnosis apparatus 100 displays a first marker 304 and a second marker 306 on the first ultrasound image 310 and the second ultrasound image 320, respectively. The scan line 303 of the first ultrasound image 310 and the depth axis 309 of the second ultrasound image 320 match with each other, and thus the first marker 304 may be displayed on the scan line 303. Meanwhile, the second marker 306 may be displayed on a 2D coordinate of the second ultrasound image 320.

If an external input signal for moving the first marker 304 to a point 305 is received using various input units such as a touch screen, a track ball, etc. and various input methods such as a drag and drop input, a touch input, etc., the ultrasound diagnosis apparatus 100 may move the first marker 304 and display a new first marker 305. The first marker 304 may move along the scan line 303.

Simultaneously with the movement of the first marker 304, the ultrasound diagnosis apparatus 100 may move the second marker 306 and display a new second marker on the point 307. In this regard, the second marker 306 may move along the depth axis 309 based on the fact that the first ultrasound image 310 and the second ultrasound image 320 match with each other. In other words, a movement distance of the second marker 306 along the depth axis 309 may be determined based on a movement distance of the first marker 304 in the scan line 303.

Also, the movement distances of the first marker 304 and the second marker 306 may mean depth values of the first ultrasound image 310 and the second ultrasound image 320. That is, the longer the movement distances of the first marker 304 and the second marker 306, the greater the change in the depth values. According to such an embodiment, although two or more ultrasound images are not compared to each other using markings, etc., markers that move by matching with each other may be used to easily measure locations and depth values in the ultrasound images.

To the contrary to the above-described embodiment, an external input signal for moving the second marker 306 to the point 307 may be received. In this regard, the ultrasound diagnosis apparatus 100 may move the second marker 306 and display a new second marker on the point 307. Unlike as shown in FIG. 3, the new second marker may not move in a vertical direction (to the point 307) but may move in a diagonal direction on a 2D coordinate plane.

Simultaneously with the movement of the second marker 306, the ultrasound diagnosis apparatus 100 may move the first marker 304 and display the new first marker 305. That is, the ultrasound diagnosis apparatus 100 may move the second marker 306 and the first marker 304 together according to a matching relationship between the first ultrasound image 310 and the second ultrasound image 320.

Since the first marker 304 moves on the scan line 303, in a case where the second marker 306 moves in the diagonal direction, time axis information in a horizontal direction in which the second marker 306 moves is not considered with respect to the movement of the first marker 304. That is, the ultrasound diagnosis apparatus 100 may move the first marker 304 on the scan line 303 according to the movement distance of the second marker 306 in a direction of the depth axis 309. Although a movement distance of the second marker 306 in the horizontal direction is not considered with respect to the movement of the first marker 304, a horizontal line 308 acting as a visual aid may be displayed on the second ultrasound image 320.

According to another embodiment, the ultrasound diagnosis apparatus 100 may display a measurement value, in addition to an ultrasound image and a marker. That is, although not shown in FIG. 3, the ultrasound diagnosis apparatus 100 may display coordinate values indicating locations of the first marker 304 and the second marker 306 in a partial region of the screen 300, and depth values obtained by measuring the movement distances of the first marker 304 and the second marker 306. In addition to the above-described measurement values, various measurement values obtained through the ultrasound image may be displayed.

According to another embodiment, to easily search for an ultrasound image, the ultrasound diagnosis apparatus 100 may display a search bar. That is, the ultrasound diagnosis apparatus 100 may display the search bar including identifiers of various forms such as "▷", "∥", "≪", "≫", etc. to allow a user to easily search for the ultrasound image.

For example, the ultrasound diagnosis apparatus 100 may display a previous or next ultrasound image from among a plurality of ultrasound images through the identifiers "≪"and "≫", and may reproduce or stop displaying an ultrasound image in a motion image through the identifiers "▷"and "∥". The above-described embodiment is merely an example. It will be understood by those of ordinary skill in the art that various auxiliary identifiers may be displayed in order to display an ultrasound image.

FIG. 4 shows an embodiment of a volume data image 420 and a planar image 410.

The ultrasound diagnosis apparatus 100 displays the volume data image 420 that is a 3D image and the planar image 410 obtained by cutting the volume data image 420. The planar image 410 may be one of an A plane image, a B plane image, and a C plane image obtained by cutting the volume data image 420 in previously determined directions. In the embodiment of FIG. 4, the A plane obtained by cutting the volume data image 420 in a horizontal direction is the planar image 410.

Also, the ultrasound diagnosis apparatus 100 may display the planar image 410, the volume data image 420, and markers including the first marker 411 and the second marker 421, as well as a cutting surface 423 for obtaining the planar image 410 by cutting the volume data image 420 in a previously determined direction on the screen 300.

The first marker 411 is displayed on the planar image 410. The second marker 421 is displayed on the volume data image 420. Since the planar image 410 is a plane obtained by cutting the volume data image 420, the second marker 421 is displayed on the cutting surface 423 of the volume data image 420 corresponding to the planar image 410.

Likewise as described with reference to FIG. 3, the ultrasound diagnosis apparatus 100 receives an external input signal using various input units such as a touch screen, a track ball, etc. and various input methods such as a drag and drop input, a touch input, etc.

If an external input signal for moving the first marker 411 to a point 412 is received, the ultrasound diagnosis apparatus 100 may move the first marker 411 and display a new first marker on the point 412.

Simultaneously with the movement of the first marker 411, the ultrasound diagnosis apparatus 100 may move the second marker 421 and display a new second marker on the point 422. In this regard, the new second marker on the point 422 may move on the cutting surface 423 of the volume data 420 corresponding to the planar image 410, based on the fact that the planar image 410 is obtained by cutting the volume data image 420.

According to another embodiment, a path in which the new second marker 422 moves on the volume data image 420 may not be the cutting surface 423. That is, in a case where the new second marker 422 is displayed in a location other than the cutting surface 423 of the volume data image 420, the first marker 411 may not be displayed on the planar image 410.

In other words, only when the new second marker on the point 422 is disposed on the cutting surface 423, may the first marker 411 be displayed on the planar image 410. Contrary to the above-described embodiment, an external input signal for moving the second marker 421 to the point 422 may be received. In this regard, the ultrasound diagnosis apparatus 100 may move the second marker 421 and display the new second marker on the point 422.

Simultaneously with the movement of the second marker 421, the ultrasound diagnosis apparatus 100 may move the first marker 411 and display the new first marker 412. That is, the ultrasound diagnosis apparatus 100 may move the second marker 421 and the first marker 411 together based on the fact that the planar image 410 is obtained by cutting the volume data image 420.

FIG. 5 shows an embodiment of a volume data image 540 and a plurality of planar images 510, 520, and 530.

FIG. 5 shows the embodiment of the volume data image 540 that is volume data and the planar images 510, 520, and 530 obtained by cutting the volume data image 540 in three different directions. The planar images 510, 520, and 530 may be, respectively, the A plane image 510, the B plane image 520, and the C plane image 530 of the volume data image 540.

The ultrasound diagnosis apparatus 100 displays the A plane image 510, the B plane image 520, and the C plane image 530 of the volume data image 540 that are the three planar images.

Thereafter, the ultrasound diagnosis apparatus 100 displays first markers 501, 502, and 503 on the A plane image 510, the B plane image 520, and the C plane image 530, respectively. Also, the ultrasound diagnosis apparatus 100 displays second markers 541, 542, and 543 matching with the first markers 501, 502, and 503, respectively, on the volume data image 540.

According to an embodiment, the first markers 501, 502, and 503 displayed on the planar images 510, 520, and 530, respectively, and the second markers 541, 542, and 543 displayed on the volume data image 540 may be visually distinguished and displayed.

That is, as shown in FIG. 5, the first marker 501 may be displayed as "○" on the A plane image 510, the first marker 502 may be displayed as "X" on the B plane image 520, and the first marker 503 may be displayed as "▲" on the C plane image 530, which are visually distinguished from each other. Also, the second markers 541, 542, and 543 that are matching with the first markers 501, 502, and 503, respectively, and displayed on the volume data image 540, may be visually distinguished and displayed like the first markers 501, 502, and 503.

According to the present embodiment, the three planar images 510, 520, and 530 with which the second markers 541, 542, and 543 displayed on the volume data image 540 match may be easily distinguished.

Although not shown in FIG. 5, the ultrasound diagnosis apparatus 100 may move one of the displayed first markers 501, 502, and 503 and second markers 541, 542, and 543 based on an external input signal. The external input signal may be received through a track ball or a touch screen. In a case where the external input signal is received through the touch screen, various types of external input signals such as a touch input, a drag and drop input, etc. may be received.

An embodiment in which the ultrasound diagnosis apparatus 100 may move the first markers 501, 502, and 503 and the second markers 541, 542, and 543 together based on the fact that the planar images 510, 520, and 530 are obtained by cutting the volume data image 540 is the same as described with reference to FIG. 4.

According to an embodiment, the second markers 541, 542, and 543 may move on a cutting surface of the volume data image 540 corresponding to each of the planar images 510, 520, and 530. That is, the ultrasound diagnosis apparatus 100 may display one or more cutting surfaces from which the planar images 510, 520, and 530 are obtained in previously determined directions, and may move each of the second markers 541, 542, and 543 on a corresponding cutting surface.

For example, the second marker 541 matching with the A plane image 510 may move on a cutting surface of the volume data image 540 corresponding to the A plane 510. Likewise, the second marker 542 matching with the B plane image 520 may move on a cutting surface of the volume data image 540 corresponding to the B plane 520, and the second marker 543 matching with the C plane image 530 may move on a cutting surface of the volume data image 540 corresponding to the C plane image 530.

According to another embodiment, a path in which the second markers 541, 542, and 543 move on the volume data image 540 may not be the cutting surface from which each of the planar images 510, 520, and 530 is obtained. That is, in a case where the second markers 541, 542, and 543 are displayed in locations other than the cutting surface from which each of the planar images 510, 520, and 530 is obtained, the first markers 501, 502, and 503 may not be displayed on the planar images 510, 520, and 530.

In the present embodiment, like as described with reference to FIG. 4, only when the second markers 541, 542, and 543 are disposed on the cutting surfaces, may the first markers 501, 502, and 503 be displayed on the planar images 510, 520, and 530.

Meanwhile, in a case where one of the second markers 541, 542, and 543 is displayed on a point where the planar images 510, 520, and 530 cross each other, a plurality of first markers may correspond to the one of the second markers 541, 542, and 543 displayed on the crossing point. In other words, a plurality of first markers may correspond to one second marker.

That is, in a case where a second marker is displayed on a point where the A plane image 510 and the B plane image 520 cross each other on the volume data image 540, two first markers corresponding to the second marker may be simultaneously displayed on the A plane image 510 and the B plane image 520. Likewise, three first markers may be displayed with respect to a second marker displayed on a point where the A plane image 510, the B plane image 520, and the C plane image 530 cross each other.

According to an embodiment, the ultrasound diagnosis apparatus 100 adjusts transparency of the volume data image 540 that is volume data and displays the volume data image 540, and thus a user may easily recognize cutting surfaces on which the second markers 541, 542, and 543 displayed on inner points of the volume data image 540 move.

According to an embodiment described with reference to FIGS. 4 and 5 above, a user of the ultrasound diagnosis apparatus 100 may exactly determine corresponding locations between ultrasound images when diagnosing an object through volume data and a planar image.

The embodiments of the present invention may be written as computer programs and may be implemented in general-use digital computers that execute the programs using a computer readable recording medium. In addition, a structure of data used in the above-described method may be recorded in a computer readable recording medium through various methods. Examples of the computer readable recording medium include magnetic storage media (e.g., ROMs, RAMs, universal serial buses (USBs), floppy disks, hard disks, etc.), optical recording media (e.g., CD-ROMs, or DVDs), and storage media such as PC interfaces (e.g., PCI, PCI-express, WiFi, etc.).

As described above, with respect to one or more ultrasound images displayed on a screen of an ultrasound diagnosis apparatus, corresponding data values between the ultrasound images may be conveniently measured. Also, a user may intuitively and exactly compare locations of the corresponding data values.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An ultrasound image displaying method comprising:
displaying a first ultrasound image obtained from an object in a first mode and a second ultrasound image obtained from the object in a second mode;
displaying a first marker and a second marker indicating locations of parts of the object on the first ultrasound image and the second ultrasound image, respectively; and
as the first marker moves based on an external input signal, moving the second marker such that the second marker matches with the moved first marker.

2. The ultrasound image displaying method of claim 1, wherein the first mode comprises a brightness (B) mode, and the second mode comprises a motion (M) mode.

3. The ultrasound image displaying method of claim 1, wherein a depth axis of the second ultrasound image matches with one of scan lines included in the first ultrasound image.

4. The ultrasound image displaying method of claim 3, wherein the external input signal moves a depth value of the first marker,
wherein the moving of the second marker comprises: moving a depth value of the second marker such that the depth value of the second marker matches with the moved depth value of the first marker.

5. The ultrasound image displaying method of claim 1, wherein the first ultrasound image is a two-dimensional (2D) image or a three-dimensional (3D) image obtained by scanning the object.

6. An ultrasound diagnosis apparatus for displaying an ultrasound image, the apparatus comprising:
an ultrasound image obtaining unit for obtaining a first ultrasound image from an object in a first mode and obtaining a second ultrasound image from the object in a second mode;
a display unit for displaying the first ultrasound image and the second ultrasound image and displaying a first marker and a second marker indicating locations of parts of the object on the first ultrasound image and the second ultrasound image, respectively; and
an external input receiving unit for receiving an external input signal,
wherein the display unit, as the first marker moves based on the external input signal, moves the second marker such that the second marker matches with the moved first marker.

7. The apparatus of claim 6, wherein the first mode comprises a brightness (B) mode, and the second mode comprises a motion (M) mode.

8. The apparatus of claim 6, wherein a depth axis of the second ultrasound image matches with one of scan lines included in the first ultrasound image.

9. The apparatus of claim 8, wherein the external input signal receiving unit receives the external input signal for moving a depth value of the first marker,
wherein the display unit moves a depth value of the second marker such that the depth value of the second marker matches with the moved depth value of the first marker.

10. The apparatus of claim 6, wherein the first ultrasound image is a 2D image or a 3D image obtained by scanning the object.

11. A computer readable recording medium storing a program for executing an ultrasound image displaying method of claim 1.
